# EUROPEAN PATENT APPLICATION

(11) **EP 3 708 662 A1**
(43) Date of publication of application: **16.09.2020**
(21) Application number: 18876286.8
(22) Date of filing: 02.08.2018
(51) Int. Cl.: C12N 15/01, A61K 35/30, A61K 35/36, A61P 25/00, C12N 1/00, C12N 5/10, C12N 5/074

(54) **METHOD FOR PRODUCING CULTURED CELL, AND METHOD FOR PRODUCING THERAPEUTIC AGENT FOR SPINAL CORD INJURY DISEASE**

(30) Priority: 10.11.2017 JP 2017216849
(71) Applicant: Regenesis Science Co., Ltd., Fukuoka-shi, Fukuoka 810-0074 (JP)
(72) Inventor: NISHINA, Hiromichi, Fukuoka-shi Fukuoka 812-0069 (JP); YANAGA, Hiroko, Fukuoka-shi Fukuoka 810-0001 (JP)
(74) Representative: van Dam, Vincent
(86) International application number: PCT/JP2018/028998
(87) International publication number: WO 2019/092939

(57) **Abstract**

[Problem to Be Solved] The present invention is to provide a method of culturing a cell, which method safely increases GDNF mRNA expression without introducing a gene with a virus.

[Means to Solve the Problem] A method of producing a cultured cell comprising glial cell line-derived neurotrophic factor (GDNF) mRNA, comprising the step of culturing a human skin-derived stem cell in a serum-free medium comprising at least one of SAG, purmorphamine and sonic hedgehog (SHH) protein. The medium may further comprise B-27 supplement, a ROCK inhibitor, EGF and FGF2.

### [Sequence Listing]

SEQUENCE LISTING

<110>  Cell Aid Laboratory Co.

<120>  Method for manfacturing cultured cells and a therapeutic agent
       for spinal cord injury

<130>  17-297P

<160>  6

<170>  PatentIn version 3.5

<210>  1
<211>  18
<212>  DNA
<213>  Artificial Sequence

<220>
<223>  primer

<400>  1
atcttctttt gcgtcgcc                                                    18


<210>  2
<211>  19
<212>  DNA
<213>  Artificial Sequence

<220>
<223>  primer

<400>  2
gatgacaagc ttcccgttc                                                   19


<210>  3
<211>  20
<212>  DNA
<213>  Artificial Sequence

<220>
<223>  primer

<400>  3
cgttggaaca gaggttggag                                                  20


<210>  4
<211>  19
<212>  DNA
<213>  Artificial Sequence

<220>
<223>  primer

<400>  4
tcctgaaagc tgagggaag                                                   19


<210>  5
<211>  20

<212>  DNA
<213>  Artificial Sequence

<220>
<223>  primer

<400>  5
acatcgagat cgccacctac                                                   20


<210>  6
<211>  20
<212>  DNA
<213>  Artificial Sequence

<220>
<223>  primer

<400>  6
acatcacatc cttgtgctcc                                                   20

## Description

### [Field of Invention]

The present invention relates to a method of producing a cell, which is obtained by culturing a cell taken from human skin tissue, for treating spinal cord injuries. The present invention also relates to a method of producing a therapeutic agent, which comprises the cell obtained by the aforesaid method, culture medium or secretions such as exosome, for treating spinal cord injuries.

### [Background of Invention]

Spinal cord injury is a pathology of spinal cord damages mainly resulting from spinal column damages caused by an external strong force applied on the spinal column. Similar damages are caused by an internal cause such as spinal cord tumor, hernia, or cervical spondylotic myelopathy. The estimated number of patients with spinal cord injury is 100,000 in Japan. Most of the causes of spinal cord injury are external traumas such as traffic accidents and falls from a height.

Unlike peripheral nerves, the central nervous system including the spinal cord does not restore or regenerate once it has been damaged. To regenerate a damaged spinal cord and restore functions thereof is the desire of patients with spinal cord injury, for which various studies are pursued.

Currently, a large amount of steroid is administered if it is within 48 to 72 hours immediately after the injury in the United States of America. Attempts are made, for example, to inject a cultured self bone marrow fluid in the spinal cord by Kansai Medical University in Japan and to administer hepatocyte growth factor (HFG) within 78 hours after spinal cord injury by a research group of Keio University in Japan.

In a chronic phase, rehabilitation is performed. However, the rehabilitation for spinal cord injury is not to restore lost functions. Since the nerve will not regenerate, such restoration is impossible. The purpose of the rehabilitation is "to use remaining functions so as to be capable of performing ADL (Activities of Daily Living)." Further, in the chronic phase, complications such as decubitus and urinary tract infection occur to make caring extremely difficult.

It is a trial of regenerating the nerve using a stem cell of bone marrow or a nerve that is regarded as the most promising treatment even in the chronic phase. A partial effectiveness has been reported in animal experiments; however, it is still at the stage of basic research for applying to human as a useful treatment and vigorous pursuits of the research are desired. Uses of an induced pluripotent stem cell and an embryonic stem cell are mainly being studied, but there are many problems to be solved. For example, the use of the induced pluripotent stem (iPS) cell is considered to be able to avoid immune reactions, but possibilities of carcinogenesis have been pointed out. For the embryonic stem cell, ethical issues have been pointed out in addition to immune reactions and carcinogenesis.

Despite the above, in 2010, a study was published in Japan reporting that a tumor was formed in the spinal cord of an animal with spinal cord injury by administration using an iPS cell made by genes introduction which had overcome the problems realating ethics and immune rejection (Non-patent document 1).

As of 2005, it is the sole clinical treatment performed at Capital Medical University in Beijing, China to regenerate the spinal cord by injecting nasal olfactory mucosal cells (OEG). No data verifying the effects of treatment for a long period of time that would convince researchers in the world, however, has been reported by the university and problems of severe pain, source of OEG (taken from aborted fetus), and allotransplantation still remain. In a Japanese clinical study for transplanting self nasal mucosa published in 2014, no dramatic effect (case of recovery to walk) was reported.

In October 2010, Geron Co. in the United States of America started a clinical test using ES cells on 4 patients with spinal cord injury, but announced its withdrawal in November 2011. Although the reasons are unknown, it is highly probable that a clinical effect was low, an immune rejection reaction was observed, or a teratoma was formed in the spinal cord. Masaya Nakamura, associate professor of Keio University, and his group announced a plan of starting a clinical study of iPS cell on patients with spinal cord injury in 2017 at a conference of the Japanese Society of Regenerative Medicine held in Kyoto. The subjects were the patients who were in 2 to 4 weeks after injury with an affected site where inflammation had been settled down and the site had not yet started to harden. Patients in chronic phase were not subjects.

Thus stem-cell therapy attracts attentions, but regenerative medicine products which are already on the market are only one in the United States of America and one in EU as an Advanced Therapy Medicinal Product. Currently. There is no approved regenerative medicine product for spinal cord injury.

Human skin-derived mesenchymal stem cells are difficult to induce to differentiate into nerve cells after transplantation and have high possibility to differentiate into chondrocyte or osteocyte. Brain-derived neural stem cells have a problem of low effectiveness of treatment (Non-patent document 13).

On the other hand, it has been reported that skin-derived progenitor cells are more effective for treating spinal cord injury than neural stem cells (Non-paten document 14).

Recently, Inoue and Kumagaya reported that human epidermal karatinocytes are effective for treating spinal cord injury (Non-patent document 2).

However, there is no method for identifying a marker of therapeutic effect on spinal cord and for culturing cells expressing the maker.

As cytokine, on the other hand, glial cell line-derived neurotrophic factor (GDNF) has most clearly shown its therapeutic effect on spinal cord injury (Non-patent documents 4-8).

Accordingly, attempts to make therapeutic medicine having improved therapeutic effects on spiral cord injury were made to introduce GDNF gene into cells which are expected to have therapeutic effects (Non-patent documents 8-10). As the cells into which the gene is introduced, olfactory ensheathing cells and Schwann cells are used. These cells have therapeutic effects themselves, which can be further improved.

However, a drawback of this method is a high risk of cancer development when transplanted to a living organ because of the use of a virus for introducing the gene.

On the contrary, GDNF protein itself is unstable and is difficult to handle (Non-patent document 15).

Meanwhile, GDNF is reported to be an endogenous factor necessary for restoring spinal cord injury (Non-patent document 16).

Further, GDNF is reported to be effective for suppressing allodynia, which torments patients with spinal cord injury (Non-patent documents 17 and 18).

Most treatments of spinal cord injury are for patients in chronic phase, so that a therapeutic method which is also effective in chronic phase is most desired. Fortunately, it is reported that GDNF is effective for chronic spinal cord injury (Non-patent document 19).

Therefore, Expressing GDNF in cells which are to be transplanted leads to a drastic improvement in therapeutic effects on spinal cord injury (Non-patent document 20).

### [Prior Documents]

### [Patent Documents]

[Patent Document 1] JP 2012-29684A

### [Non-patent Documents]

[Non-patent Document 1] Tsuji O, et al. : Therapeutic potential of appropriately evaluated self-induced pluripotent stem cells for spinal cord injury. PNAS 2010; 107 (28), 12704-12709
[Non-patent Document 2] Inoue H, et al. : Improvement of hind-paralysis following traumatic spinal cord injury in rats by grafting normal human keratinocytes: new cell-therapy strategy for nerve regeneration. J Artif Organs. 2011;14(4):375-80
[Non-patent Document 3] Inoue H, et al. : Improvement of hind-limb paralysis following traumatic spinal cord injury in rats by grafting normal human keratinocytes: new cell-therapy strategy for nerve regeneration. J Artif Organs. 2011 ;14(4):375-80
[Non-patent Document 4] Ansorena E, et al. : Injectable alginate hydrogel loaded with GDNF promotes functional recovery in a hemisection model of spinal cord injury. Int J Pharm. 2013 ;455(1-2):148-58.
[Non-patent Document 5] Tang XQ, et al. : Adenovirus-mediated delivery of GDNF ameliorates corticospinal neuronal atrophy and motor function deficits in rats with spinal cord injury.Neuroreport. 2004;15(3):425-9.
[Non-patent Document 6] Guzen FP, et al. : Glial cell line-derived neurotrophic factor added to a sciatic nerve fragment grafted in a spinal cord gap ameliorates motor impairments in rats and increases local axonal growth. Restor Neurol Neurosci. 2009;27(1):1-16.
[Non-patent Document 7] Kao CH, et al. : Exogenous administration of glial cell line-derived neurotrophic factor improves recovery after spinal cord injury. Resuscitation. 2008 ;77(3):395-400.
[Non-patent Document 8] Koelsch A, et al. : Transgene-mediated GDNF expression enhances synaptic connectivity and GABA transmission to improve functional outcome after spinal cord contusion. J Neurochem. 2010 ;113(1):143-52.
[Non-patent Document 9] Cao L, et al. : Olfactory ensheathing cells genetically modified to secrete GDNF to promote spinal cord repair. Brain. 2004 Mar;127(Pt 3):535-49.
[Non-patent Document 10] Deng LX, et al. : A novel growth-promoting pathway formed by GDNF-overexpressing Schwann cells promotes propriospinal axonal regeneration, synapse formation, and partial recovery of function after spinal cord injury. J Neurosci. 2013 Mar 27;33(13):5655-67.
[Non-patent Document 11] Stephen J. A. Daviees, et al. : Transplantaion of specific human astrocytes promotes functional recovery after spinal cord injury. PLoS ONE 2011;6(3): e17328
[Non-patent Document 12] Sowa NA, et al. : Ecto-5'-nucleotidase (CD73) inhibits nociception by hydrolyzing AMP to adenosine in nociceptive circuits. J Neurosci. 2010 Feb 10;30(6):2235-2244.
[Non-patent Document 13] Kim MS, et al. : Perspectives on Tissue-Engineered Nerve Regeneration for the Treatment of Spinal Cord Injury. Tissue Eng Part. 2014;20(13-14):1781-3
[Non-patent Document 14] Biemaskie J, et al. : Skin-derived precursors generate myelinating Schwann cells that promote remyelination and functional recovery after contusion spinal cord injury. J Neuroscience. 2007;27(36):9545-9559.
[Non-patent Document 15] Zhao YZ, et al. : Thermosensitive heparin-poloxamer hydrogels enhance the effects of GDNF on neuronal circuit remodeling and neuroprotection after spinal cord injury. J Biomed Mater Res A. 2017;5(10):2816-2829
[Non-patent Document 16] Zhou HL, et al. : Changes in Glial cell line-derived neurotrophic factor expression in the rostral and caudal stumps of the transected adult rat spinal cord.Neurochem Res. 2008;33(5):927-37
[Non-patent Document 17] Chou AK, et al. : Adenoviral-mediated glial cell line-derived neurotrophic factor gene transfer has a protective effect on sciatic nerve following constriction-induced spinal cord injury. PLoS One. 2014;9(3): e92264
[Non-patent Document 18] Detloff MR, et al. : Acute exercise prevents the development of neuropathic pain and the sprouting of non-peptidergic (GDNF- and artemin-responsive) c-fibers after spinal cord injury. Exp Neurol. 2014;255:38-48.
[Non-patent Document 19] Dolbeare D, et al. : Restriction of axonal retraction and promotion of axonal regeneration by chronically injured neurons after intraspinal treatment with glial cell line-derived neurotrophic factor (GDNF). J Neurotrauma. 2003;20(11):1251-61.
[Non-patent Document 20] Lu Y, et al. : Glial cell line-derived neurotrophic factor-transfected placenta-derived versus bone marrow-derived mesenchymal cells for treating spinal cord injury. Med Sci Monit. 2017 ;23:1800-1811
[Non-patent Document 21] Codega P, et al. : Prospective identification and purification of quiescent adult neural stem cells from their in vivo niche. Neuron. 2014;82(3):545-59.
[Non-patent Document 22] DeCarolis NA, et al. : In vivo contribution of nestin-and GLAST-lineage cells to adult hippocampal neurogenesis. Hippocampus. 2013;23(8):708-719.
[Non-patent Document 23] Sufan Wu, et al. : Bone marrow stromal cells enhance differentiation of cocultured neurosphere cells and promote regeneration of injured spinal cord. J Neurosci Res. 2003;72(3):343-51.

### [Summary of Invention]

### [Technical Problem]

An object of the present invention is to provide a method of culturing a cell to safely increases GDNF mRNA expression of the cell without introducing a gene with a virus.

Another object of the present invention is to provide a method of culturing a cell that expresses various markers such as a marker of neural stem cell.

Still another object of the present invention is to provide a method of producing a therapeutic agent for spinal cord injury.

### [Solution to Problem]

The present invention is based on an experimental finding that glial cell line-derived neurotrophic factor (GDNF) mRNA can be highly expressed (for example, at least 10 times expression level of the normal culture) by culturing human skin-derived stem cell in a serum-free medium comprising at least one of SAG, purmorphamine, and Sonic hedgehog (SHH) protein.

The first aspect of the present invention relates to a method of producing a cultured cell comprising glial cell line-derived neurotrophic factor (GDNF) mRNA. This method comprises the step of culturing a human skin-derived stem cell in a serum-free medium comprising at least one of SAG, purmorphamine, and Sonic hedgehog (SHH) protein. By this method, glial cell line-derived neurotrophic factor (GDNF) mRNA is highly expressed.

The human skin-derived stem cell preferably originates from a skin cell of a patient. In particular, cells to be cultured are preferably collected from the skin tissue of which damages are mild and recovery is fast. Since the methods of culturing a stem cell are known, cells may, basically, be cultured according to the method described herein or known methods (for example, Japanese Patent No.5409359 and Japanese Patent No.6041270). The method of culturing a human skin-derived stem cell is also known and described in the above patent documents.

SAG refers to a known chemical compound with CAS No.364590-63-6 of which chemical name is "N-Methyl-N'-(3-pyridinylbenzyl)-N'-(3-chlorobenzo[b]thiophene-2-carbonyl)-1,4-diaminocyclohexane."

As shown in WO 2014-084085 pamphlet, SAG is a chemical compound which activates sonic hedgehog (Shh). SAG1.1 has the chemical name of "N-Methyl-N-(3-(4-benzonitrile)-4-methoxybenzyl)-N'-(3-chlorobenzo[b]thiophene-2-carbonyl)-1,4-diaminocyclohexane." SAG is detailed in Sinha S, Chen JK. Nat Chem Biol. 2006 Jan;2(1):29-30, and Chen JK, Taipale J, Young KE, Maiti T, Beachy PA. Proc Natl Acad Sci U S A. 2002 Oct 29;99(22):14071-6. Similarly, SAG1.1 is detailed in Chen, W., Ren, X. R., Nelson, C. D., Barak, L. S., Chen, J. K., Beachy, P. A.,de Sauvage, F. & Lefkowitz, R. J. (2004) Science 306,(5705) 2257-2260.

Purmorphamine is a generic name of 9-Cyclohexyl-N-[4-(4-morpholinyl)phenyl]-2-(1-naphthalenyloxy)-9H-purin-6-amine. Purmorphamine is an Smoothened agonist and is described in Japanese Patent No. 6210881 and Japanese Patent No. 5620821 where 2 µM thereof is added to a medium.

Sonic Hedgehog (SHH) protein is a known protein and, in Japanese Patent No.4900587, for instance, 200 to 400 ng/mL of SHH protein is added to a serum-free medium.

### Serum-free medium

The medium in the present invention is a serum-free medium which may comprise a component of a known medium. For example, Japanese Patent No.4385076 and Japanese Patent Laid-open No.2012-157263 disclose a medium for culturing an animal cell in a serum-free medium. Elements described in these known documents may be added as desired to the medium in the present invention.

As a basal culture medium of the medium in the present invention, a known medium may be used. Examples of such basal medium include MEM medium, Dulbecco's MEM (registered trade name), MCDB153, Ham's F12(registered trade name) medium, McCoy's 5A (registered trade name) Medium, Medium 199 Earle's Liquid Media (registered trade name), RPMI1640(registered trade name) Medium, Ham's F10 medium, MEM-α medium, DMEM/F12medium, MCDB131, MCDB153 and MCDB201.

The medium, when it is equilibrated in an environment of 5% CO₂, has a pH adjusted in the range of from pH 6.8 to 7.8, preferably 7.2 (± 0.1). An acidity of the basal medium may be adequately controlled by using a buffer material (for example, sodium bicarbonate), or pH control agent such as hydrogen chloride or sodium hydroxide.

The medium in the present invention may comprise various low molecular weight compounds as desired. For example, the medium in the present invention preferably comprises an antioxidant. Examples of the antioxidant are one or more component selected from the group consisting of Melatonin, N-acetyl-L-cysteine, reduced type of glutathione and ascorbic acid. The medium in the present invention may comprise phospholipid (for example, phosphatidylserine, phosphatidylethanolamine, and phosphatidylcholine) and fatty acid (for example, linoleic acid, oleic acid, linolenic acid, arachidonic acid, myristic acid, palmitoyl acid, palmitic acid, and stearic acid). Examples of other components that can be added to the medium in the present invention include transferrin, selenate, glucose, D-biotin, D-pantothenic acid calcium salt, choline chloride, folic acid, myo-inositol, nicotinamide, p-aminobenzoic acid, pyridoxal hydrochloride, pyridoxine hydrochloride, riboflavin, thiamine hydrochloride, Vitamin B12, sodium pyruvate, thymidine, hypoxanthine, sodium selenite, streptomycin sulfate, Penicillin G potassium salt, and phenol red.

In the aforesaid method of producing a cultured cell, it is preferred that the serum-free medium further comprises B-27 supplement. As shown in the Examples, nestin mRNA is highly expressed when the serum-free medium comprises B-27 supplement. Nestin gene is a neural stem cell marker. B-27 supplement is known element as described in, for example, Japanese Patent No. 6185907 and Japanese Patent No. 6137626 where it is added to a medium.

In the aforesaid method of producing a cultured cell, it is preferred that the serum-free medium further comprises a ROCK inhibitor. When the serum-free medium comprises a ROCK inhibitor, stability of the stem cell is maintained. The ROCK inhibitor is defined as a substance that inhibits the kinase activity of Rho-kinase and examples thereof include Y-27632 (4-[(1R)-1-aminoethyl]-N-pyridin-4-yl-cyclohexane-1-carboxamide or dihydrochloride thereof (See, for example, Ishizaki et al., Mol. Pharmacol. 57, 976-983 (2000);Narumiya et al., Methods Enzymol. 325,273-284 (2000)), Fasudil/HA1077(1- (5-Isoquinolinesulfonyl) homopiperazine) or dihydrochloride thereof (See, for example, Uenata et al., Nature 389: 990-994 (1997)), H-1152 ((S)-(+)-2-Methyl-1-[(4-methyl-5-isoquinolinyl)sulfonyl]-hexahydro-1H-1,4-diazepine) or dihydrochloride thereof (See, for example, Sasaki et al., Pharmacol. Ther. 93: 225-232 (2002)), Wf-536 ((+)-(R)-4-(1-aminoethyl)-N-(4-pyridyl)benzamide hydrochloride (See, for example, Nakajima et al., Cancer Chemother. Pharmacol. 52(4): 319-324 (2003)) and derivatives thereof, antisense nucleic acid to ROCK, RNA interference-inducing nucleic acid (For example, siRNA), dominant negative mutant and expression vectors thereof. As ROCK inhibitor, other low molecular weight compounds are known and those compounds and derivatives thereof can be used in the present invention (See, for example, U.S. Patent Publication No.20050209261, U.S. Patent Publication No.20050192304, U.S. Patent Publication No.20040014755, U.S. Patent Publication No.20040002508, U.S. Patent Publication No. 20040002507, U.S. Patent Publication No.20030125344, U.S. Patent Publication No.20030087919, and WO2003/062227, WO 2003/059913, WO 2003/062225, WO 2002/076976, WO 2004/039796). In the present invention, at least one ROCK inhibitor can be used. The ROCK inhibitor is generally used in an amount of from 100 nM to 50 µM.

In the aforesaid method of producing a cultured cell, it is preferred that the serum-free medium further comprises EGF. When the serum-free medium comprises EGF, neural stem cells are maintained and Nestin mRNA is highly expressed.

EGF is a growth factor selected from Epidermal Growth Factor (EGF) family. For example, in Japanese Patent No.6191694, EGF is contained in a medium at a final concentration ranging from 0.5 to 200 ng/mL.

GFAP gene is a marker of adult neural stem cell or astrocytes, which are considered to be suitable for transplanting to a spinal cord injury site (Non-Patent Literatures 1, 20, 22).

In the aforesaid method or producing a cultured cell, it is preferred that the serum-free medium further comprises FGF2. As shown in Examples, EGF mRNA is highly expressed and CD 73 protein is expressed when the serum-free medium contains the ROCK inhibitor. FGF (Fibroblast Growth Factor) 2 is designated also as FGF-2. For example, in Japanese Patent No.5856029, FGF2 is added to a medium at a concentration ranging from 0.1 to 50 ng/mL, more preferably from 1 to 10 ng/mL, and most preferably from 5 ng/mL. CD73 (Ecto-5'-nucleotidase) is regarded to highly possibly relieve a sharp pain peculiar to spinal cord injury patients.

In the aforesaid method of producing a cultured cell, the serum-free media preferably contains calcium ion at a concentration ranging from 0.03 mM to 0.12mM. By keeping the calcium concentration in the range, a differentiation of the stem cell can be suppressed.

The preferred embodiments mentioned above can be appropriately combined with each other. The aforesaid additives such as SAG, Purmorphamine, Sonic Hedgehog (SHH) protein, B-27 supplement, ROCK inhibitor, EGF, and FGF2 may be added to the medium in an appropriate amount according to properties of the additive. Since these substances are known to be added to a medium, an amount of addition thereof may be adjusted according to known documents. For example, each additive may be added in an amount ranging from 0.1 ng/mL to 20 µg/mL (or 0.2 ng/mL to 10 µg/mL). These additives may be added according to a degree of purity and desired amount.

In the aforesaid method of producing a cultured cell, the human skin-derived stem cell is preferably the one obtained by treating collected human skin with dispase, trypsin, and collagenase in this order. The dispase treatment, trypsin treatment, and collagenase treatment are so-called enzyme treatments. The enzyme treatments can be performed in an isotonic salt solution buffered to a physiologically acceptable pH, for example, of about 6 to 8, preferably from 7.2 to 7.6 such as PBS or Hank's balanced salt solution for trypsin and MEM medium for dispase and collagenase at a temperature ranging from about 4 to 40 °C, preferably about 4 to 39 °C, for a time sufficient to decompose connective tissues, for example, about 1 to 1,000 minutes, preferably from 5 to 720 minutes and at a sufficient enzyme concentration, for example, ranging from about 0.0001 to 5% w/v, preferably from about 0.001 to 0.5% w/v.

The second aspect of the present invention provides a method of producing a therapeutic agent for spinal cord injury. The spinal cord is the central nervous system passing through the spine and it is the therapeutic agent for spinal cord injury that is used to heal this nerve. In the method, a cultured cell is produced by using the aforesaid method. Subsequently, the therapeutic agent for spinal cord injury (hereinafter may be referred to as the agent of the present invention) is produced, which agent comprises, as an active ingredient, one or more of the cultured cell, a medium obtained in the culture step, and a secretion obtained in the culture step (such as exosome). The secretion (such as exosome) may originate from a culture supernatant. As a medium, the culture supernatant may be solely used.

The agent in the present invention may be prepared by a method known in the art. The agent in the present invention may be formulated as an oral agent or a parenteral agent, preferably parenteral agent. Such parenteral agent may be formulated as a liquid agent (such as aqueous liquid agent, non-aqueous liquid agent, suspension agent, or emulsion agent) or a solid agent (such as solidified powder agent, or freeze-dried agent). The agent in the present invention may be formulated as a sustained release agent. A dosage form of the agent is preferably liquid when the agent comprises living cells and a dosage form of the agent can either be liquid or solid when the agent comprises a part of a cell or dead cells as a whole.

A major component of the agent in the present invention is one or more of cultured human skin-derived stem cell, a medium obtained in the culture step, and secretions of the cultured cells (such as exosome). In any case, an effective dose may be designed by using the number of cells as an index, for instance. A final agent may be devoid of the stem cell. The secretions may be those contained in a supernatant of a culture medium. A dose, the number of administrations, and frequency of the administration vary according to subject, age thereof, symptoms thereof or the like. Generally, an example of dose of the agent comprising human skin-derived stem cells per day as one administration of intravenous or intrathecal injection as a unit ranges from 1×10⁵ to 1×10⁹ cells, preferably from 1×10⁶ cells to 5×10⁸ cells, more preferably from 1×10⁷ cells to 2×10⁸ cells. Alternatively, an example of a dose per kg of body weight per day of the human skin-derived stem cell ranges from 1×10⁴ cells to 1×10⁸ cells, preferably from 1×10⁵ cells to 5×10⁷cells, more preferably from 1×10⁶ cells to 2×10⁷ cells. The agent in the present invention may be administered once in every 3 days, 8 administrations in total, or administered once a day for one week. Combinations of various frequencies with periods of administration can be employed. In the case of complete response where significant improvements of symptoms are observed, only an initial one administration may be sufficient. Thus, the agent in the present invention can be used in various manners according to diseases and symptoms, and the number of cells to be transplanted for each dose can be determined as desired in the aforesaid ranges.

The present invention further provides a use of the human skin-derived stem cell in the present invention as described above for producing the therapeutic agent for spinal cord injury.

The present invention also provides a method of treating spinal cord injury. The treating method comprises the step of administering an effective amount of the agent in the present invention produced in the manner as described above to a subject with spinal cord injury.

### [Advantageous Effects of Invention]

The present invention can provide a method of culturing a cell to safely promote expression of GDNF gene (mRNA) without employing a method of gene introduction using virus.

The present invention can provide a method of culturing a cell to express various markers such as a marker of neural stem cell.

The present invention can provide a method producing a therapeutic agent for spinal cord injury.

In the method of the present invention, cells for treating spinal cord injury can be obtained from the human skin, which is particularly easy to collect among human tissues, by a serum-free and feeder-free culturing method. In the method of present invention, a method has been found to increase by 10 times expression of GDNF, which is the most required element for treating spinal cord injury, and it has also been confirmed that cells highly effective for treating spinal cord injury can be obtained through expression of nestin, which is a marker of neural stem cell, expression of GFAP, which is effective for treating spiral cord injury, and expression of CD73, which prevents hyperalgesia that occurs in spinal cord injury, without introducing genes. The cell obtained by the present method showed therapeutic effect in a rat spinal cord injury model. The cell showed negative results in a carcinogenicity test. A 47-year old patient with Th3 spinal cord injury, who had not been able to walk for more than 2 years, received a twice a month-administration of the cell by lumber puncture. He became to walk one month after the administration. On the other hand, in the TRI symposium in 2014, no case of recovery to walk in four cases of age 35 or lower with complete leg paralysis (Th 4-7) was reported by nasal mucosa transplantation performed in Japan.

### [Brief Description of Drawings]

[Fig.1] Fig.1 is a graph as a drawing, which graph shows an effect of increase in GDNF mRNA expression (Example 2).
[Fig.2] Fig.2 is a graph as a drawing, which graph shows expression of Nestin mRNA (Example 3).
[Fig.3] Fig.3 is a graph as a drawing, which graph shows expression of GFAP mRNA (Example 4).
[Fig.4] Fig.4 is a graph as a drawing, which graph shows expression of CD73 (CD expression profile) (Example 5).
[Fig.5] Fig.5 is a graph as a drawing, which graph shows effects on rat spinal cord injury models (BBB score) (Example 6).
[Fig.6] Fig.6 is a graph as a drawing, which graph shows an evaluation of carcinogenicity.

### [Description of Embodiments]

The embodiments for carrying out the present invention are explained below. The present invention is not limited to the embodiments explained below and include embodiments obvious therefrom appropriately modified by a person having ordinary skills in the art based on the following embodiments.

The present invention presents a method of providing a cell for direct administration by obtaining the cell that is highly effective for spinal cord injury through the steps of:
collecting a tissue from human skin and sterilizing the tissue;
collecting a cell and seeding the cell;
proliferating the cell;
subculturing the cell;
inducing and differentiating the cell;
transplanting and processing the cell.

The present invention presents a method of providing a cell combined with other base material for medical instruments. The present invention also presents a method of administering a component of a medium used for culturing in the aforesaid steps.

Each step will be explained in detail with reference to examples. The explanation is an example and should not be considered to limit the present invention. Embodiments obvious therefrom appropriately modified by a person having ordinary skills in the art based are also encompassed in the invention. In the following, "u" may represent "micro."

### (Step of collecting tissue)

Skin collected in this step is preferably collected from or above the neck and, from the cosmetic view point after the collection, skin from a postauricular region is most preferred. Gender is not limited and age up until 70's is allowed. Subsequent steps are performed with aseptic manipulation. The collected tissue is sterilized with 70% ethanol aqueous solution that has been treated with a sterilizing filter having a pore size of 0.1 um to remove bacterial spores.

### (Step of collecting cell and seeding cell)

The tissue cut into pieces were placed in a dispase solution (PBS(-)) comprising 10uM Y-27632 at 4°C overnight. The tissue is then placed in a Trypsin solution (PBS(-)) comprising 10uM Y-27632 at 37 °C for 20 minutes. Subsequently, the tissue is soaked in a collagenase solution (DMEM) to digest the skin at 37 °C, while stirring for 1 to 2 hours. The suspension thus obtained is then filtered through a 100um mesh to remove residues. The filtrate is then subjected to centrifugation followed by washing with PBS(-) for 3 times to remove enzymes. The cells thus obtained are placed in Trypsin Inhibitor and are allowed to stand for 10 minutes. After removing the Inhibitor by centrifugation, the cells are suspended in a medium comprising 10uM Y-27632 and then are seeded in a culture dish.

### (Step of proliferating cells)

Until 6 days after seeding, the medium shall only be added, but not be changed. Subsequent medium change is carried out by recovering the cells proliferated and suspended in a conditioned medium by centrifugation, and then returning the cells suspended in a new medium to a culture system.

### (Step of subculturing cells)

The cells are subjected to a 10uM Y-27632 treatment for more than 30 minutes and are recovered from the dish with EDTA and TrypLEx Express. The cells are then placed in Trypsin Inhibitor and are allowed to stand for 10 minutes. After removing the Inhibitor by centrifugation, the cells are suspended in a medium comprise ng 10uM Y-27632, which is seeded in a dish having an area 4 or 5 times as large as that of the previously used dish. A primary cell culture is performed using 5 dishes each having an area of 100 mm². When the culture reaches to a confluence, the cells of 3 dishes may be used for transplantation or be cryopreserved until transplantation. The cells of the remaining 2 dishes are subjected to subculture using 6 dishes each having an area of 100 mm² and, when the subculture reaches to a confluence, the cells are subjected to the step of adding factors to induce.

### (Step of adding factors to induce)

The step of adding factor for induction is a step where cells are treated with an agent 3 days before transplantation and cultured by adding necessary factors. Using this step, a cell highly expresses GDNFmRNA, for example, 10 times as high as that of ordinarily cultured cell, can be obtained. Using this step, a CD13 negative cell (CD13(-)) can be obtained. Using this step, CD34 negative cell (CD34(-)) can be obtained. Using this step, CD45 negative cell (CD45(-)) can be obtained. Using this step, CD90 negative cell (CD90(-)) can be obtained. Using this step, CD73 positive cell (CD73(+)) can be obtained. A ratio (number of cells) of CD73 positive cell to cultured cells may vary from 20 % to 100%, 40% to 100%, 60% to 99%, or 80 % to 99%, for instance. The cells that have undergone the step adding factors to induce are preferably transplanted after 3 days or cryopreserved until transplantation.

### (Step of transplanting or processing)

A sufficient number of cells for treatment obtained by the above steps are treated with 10uM Y-27632 and are recovered from the dish using EDTA and TrypLEx Express. The cells are then placed in Trypsin Inhibitor and are allowed to stand for 10 minutes. After removing the Inhibitor by centrifugation, the cells are suspended at a concentration of 10⁷ cell/mL in an infusion solution when they are to be transplanted by spinal puncture. The cells may be subjected to processing with other base materials or other cells.

The present invention will be explained with reference to the following Examples. The present invention encompasses embodiments which a person having ordinary skills in the art can conceive using known technologies based on the following Examples.

### [Example 1]

### (Collection and culture of human skin-derived stem cell)

### (Collection of tissue)

All layers of about 2-cm²-wide skin excised from a face of 56-year-old female in her face-lift cosmetic surgery were used. The subsequent steps were all performed with aseptic manipulation. This collected tissue was soaked for 30 seconds in a 70% ethanol aqueous solution that has been sterilized with a filter having a pore size of 0.1 um (micron), and immediately washed with a Phosphate Buffer Saline (PBS(-)) for three times to remove ethanol. From the tissue, subcutaneous fatty tissue and dermal connective tissue were removed as much as possible with a scissor, leaving sites where hair bulbs exist, which was then cut into about 1-mm²-wide pieces with a scalpel.

### (Step 1 of collecting and seeding cells)

The pieces of the tissue were soaked overnight in 5 mL of a pre-cooled Dispase solution (25 units/ml of PBS(-)) containing 10uM Y-27635 at 4 °C.

### (Step 2 of collecting and seeding cells)

This was centrifuged to remove the supernatant. The remaining tissues were then soaked in a TrypLE Express solution (diluted 5x in PBS(-)) at 37°C for 20 minutes.

### (Step 3 of collecting and seeding cells)

This tissue was centrifuged to remove the supernatant. The remaining tissues were then soaked in 20 mL of collagenase solution (GIBCO: type I) (1400 units/ mL DMEM medium) at 37°C, while stirring for 1.5 hours.

### (Step 4 of collecting and seeding cells)

The suspension after stirred was passed through 100 um mesh to remove undigested residue. The liquid thus obtained was washed with PBS(-) followed by centrifugation to remove enzymes, which process was repeated for three times, and cells were obtained.

### (Step 5 of collecting and seeding cells)

The cells were placed in 5 mL of Soybean Trypsin Inhibitor (0.25% in PBS(-)) and were allowed to stand for 10 minutes.

### (Step 6 of collecting and seeding cells)

After removing the Inhibitor by centrifugation, the cells were suspended in a medium containing 10uM Y-27632 and then seeded in a culture dish. The cells of starting tissue of 1 to 2-cm²-wide were seeded in all the wells of a 6-well plate (CellBind: COSTER, trade name).

### (Step of preparing a medium)

A medium was prepared as follows. That is, insulin (10mg/L), holo-transferrin (5.5mg/L), selenium (6.7ug/L), ethanolamine (2mg/L), Vitamin C (L-Ascorbic acid 2-phosphate semimagnesium salt) (50ug/L), KGF (10ug/L), lipid (fatty acid mixtures: arachidonic acid (20ug/L), cholesterol (2.2mg/L), DL-a-Tocopherol-acetate (700ug/L), linoleic acid (100ug/L), linolenic acid (100ug/L), myristic acid (100ug/L), oleic acid (100ug/L), palmitoleic acid (100ug/L), palmitic acid (100ug/L), stearic acid (100ug/L), Tween 80 (22mg/L), Pluronic F-68 (1000mg/L), EGF (20ug/L), FGF (10ug/L),Y-27632 (1uM), and 1%B-27 supplement XenoFree CTS (Invitrogen) were added in MCDB 153 medium (Sigma, Stemline Keratinocyte Basal Medium) which is a basal medium.

### (Step 1 of cell proliferation)

The primary culture was performed in a conditioned medium by adding a 1/3 of the medium without changing the medium for initial 7 days. Subsequent culture was performed by changing the medium every 2 days. The change of the medium was performed by centrifuging and discarding 2/3 of the used medium, and then adding a fresh medium in an amount of 2/3 volume of the medium to the mixture of the remaining 1/3 of the used medium and floating cells precipitated by the centrifugation, which was placed back in a culture dish. It took 10 days to reach 100 % confluence.

### (Step of subculturing cells)

Y-27632 was added to the medium, where the cells had been cultured, at a concentration of 10 uM and allowed it to stand at 37 °C for 30 minutes. Then, the medium was entirely removed and 0.05%EDTA/PBS(-) was added in an amount of 2 ml/well, and allowed it to stand at 37 °C for 10 minutes. Subsequently, TrypLEx Express diluted by 5 times with PBS(-) was added in an amount of 0.5 ml/well and allowed it to stand at 37 °C for 5 minutes. The cells were detached from the wells by pipetting. Trypsin Inhibitor (0.05%/PBS(-)) was added in an amount of 2.5 ml/well to the recovered cells and then centrifugated. After removing a supernatant, 2.5 mL of Trypsin Inhibitor (0.25%/PBS(-)) was added to suspend the cells and allowed it to stand for 10 minutes. After removing the Inhibitor by centrifugation, the cells were suspended in a medium containing 10 uM Y-27632 and seeded in 5 sheets of 6-well plate (CellBind :COSTAR) having an area 5 times as large as that of the previously used one. 100 % confluence was reached after one week, and the cells thus obtained were used for following experiments.

### [Example 2]

### (A method of increasing expression of Glial cell-Derived Neurotrophic Factor (GDNF) mRNA)

To find a compound or cytokine to increase the expression of GDNF in cells, following 6 candidate materials were examined: Valproic Acid (VA), Platelete-Derived Growth Factor (PDGF), SAG (or Purmorphamine or rh-Sonic Hedgehog (PeproTech)), Bone Morphogenic Protein 4 (BMP4). These 6 materials were added to 7 wells, which had reached to confluence in Example 1, in the following combinations: (1) Control (not added) (2) VA (1uM), (3) VA (1uM) + PDGF (10ng/ml), (4) VA (1uM) + SAG (0.3uM), (5) PDGF (10ng/ml), (6) SAG (0.3uM), (7) MBP-4 (10ng/ml). 72 hours after the addition, total RNA was extracted from each well by TRIzol method. Using "ReverTra Ace (trade mark) qPCR RT kit", a cDNA synthesis kit for PCR, 0.5 ug of each total RNA was added to 10 uL of ReverTra Ace (trade mark) qPCR RT kit to synthesize respective cDNA.
Expression of GDNFmRNA in these cDNA samples was compared by real-time PCR method using a GDNF real-time PCR primer set (Roche 5326257) and by intercalator assay using ABI PRISM (trade mark) 7700 of Applied Biosystems Inc. For a qRT-PCR reaction, SYBR (trade name) Green Realtime PCR Master Mix -Plus- from TOYOBO Co.Ltd. was used to prepare a reaction solution.

**Preparation of the reaction solution**

| | |
|---|---|
| Distilled water | 11 µL |
| SYBR (trade mark) Green Realtime PCR Master Mix -Plus- | 25 µL |
| Plus solution | 5 µL |
| Primer 1(10 µM) | 2 µL |
| Primer 2(10 µM) | 2 µL |
| Sample solution | 5 µL |
| Total volume | 50 µL |

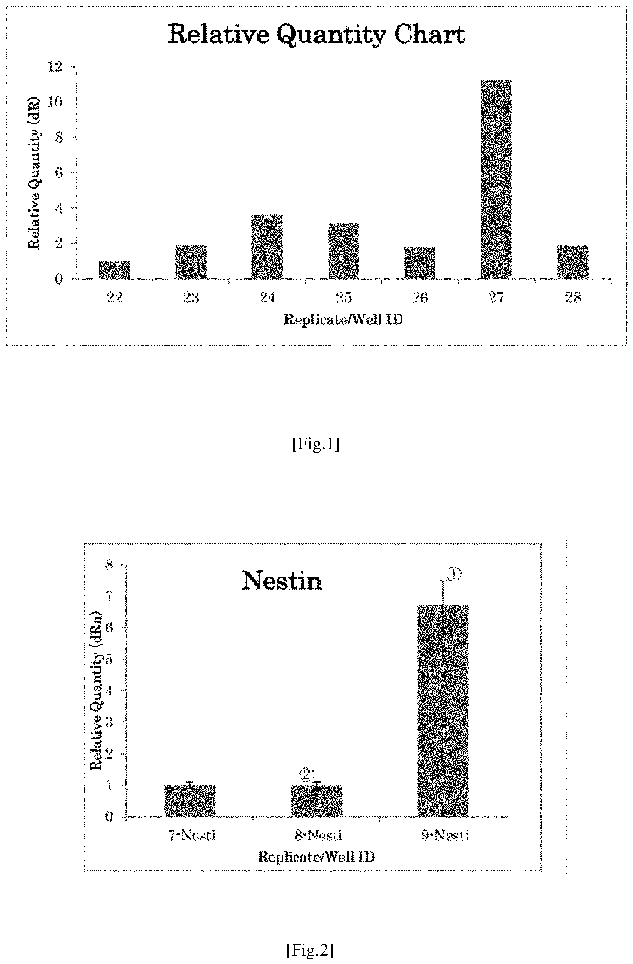

The reaction solution was placed in each well of a 96-well plate where the PCR reaction was carried out.
PCR was performed by three-step method under the conditions of annealing 60°C/elongation 72°C and the steps (1) to (2) were repeated for 40 cycles. In the last step, a Melting Curve was analyzed and it was confirmed that no primer dimer was produced.
95°C, 60 seconds
(1)↓
(2)95°C, 15 seconds
(3)60°C, 15 seconds
(4)72°C, 45 seconds (data collection)
Detection of GAPDHmRNA was performed simultaneously, and the expression of GDNFmRNA was corrected to GDNFmRNA/ GAPDHmRNA. As the result, (6) SAG only achieved more than 10 times as much expression of GDNFmRNA as that of (1) control. Purmorphamine (1.5uM) and rh-Sonic Hedgehog(10ng/ml) used in place of SAG also achieved nearly 10 times as much expression of GDNFmRNA (Fig.1). GAPDH was synthesized using the following primers:
Forward primer: ATCTTCTTTTGCGTCGCC (SEQ ID No.1)
Reverse primer: GATGACAAGCTTCCCGTTC (SEQ ID No.2)
Expression chain length: 250bp

Fig.1 is a graph as a drawing, which shows the results of GDNF mRNA expression in Example 2.

### [Example 3]

### (Change in expression by induced neuronal differentiation of Nestin mRNA)

Among 3 wells, which reached to confluence in Example 1, the cells of 2 wells were cultured for 7 days in a medium for NPC differentiation for identifying differentiated neural progenitor cells of Human/Mouse/Rat Neural Progenitor Cell Functional Identification Kit (R&D Systems SC082). The cells of the other one well were cultured in a maintenance medium of the present invention for the same period of time. On the 7th day of the culture, total RNA was extracted, and cDNA was prepared in the same manner as in Example 2. Using the cDNA, expression of Nestin mRNA, which is a marker of the neural stem cell, was measured by RT-PCR and compared with GAPDH mRNA as the basal expression. Primers for Nestin were as follows:
Forward primer: CGTTGGAACAGAGGTTGGAG(SEQ ID No.3)
Reverse primer:TCCTGAAAGCTGAGGGAAG(SEQ ID No.4)
Expression chain length: 262bp

The same primers as those used in Example 2 were used for GAPDH.

Results are shown in Fig.2.

Fig.2 is a graph as a drawing, which shows the expression of Nestin mRNA in Example 3. As shown in Fig.2, the expression of Nestin (Nestin mRNA/GAPDH mRNA) decreased to about 1/7 of the level before the differentiation by changing a culture medium to the differentiation medium. This result indicates that the proliferation medium of the present invention maintains neural stem cells, whereas the differentiation medium decreases neural stem cells.

### [Example 4]

### (Change in expression by induced neuronal differentiation of GFAP mRNA)

Among 3 wells, which reached to confluence in Example 1, the cells of 2 wells were cultured for 7 days in a medium for NPC differentiation for identifying differentiated neural progenitor cells of Human/Mouse/Rat Neural Progenitor Cell Functional Identification Kit (R&D Systems SC082). The cells of the other one well were cultured in a maintenance medium of the present invention for the same period of time. On the 7th day of the culture, total RNA was extracted, and cDNA was prepared in the same manner as in Example 2. Using the cDNA, expression of GFAP mRNA, which is a marker of astrocytes or adult neural stem cells, was measured by RT-PCR and compared with GAPDH mRNA as the basal expression. Primers for GFAP were as follows:
Forward primer: ACATCGAGATCGCCACCTAC(SEQ ID No.5)
Reverse primer: ACATCACATCCTTGTGCTCC (SEQ ID No.6)
Expression chain length: 219bp

The same primers as those used in Example 2 were used for GAPDH.

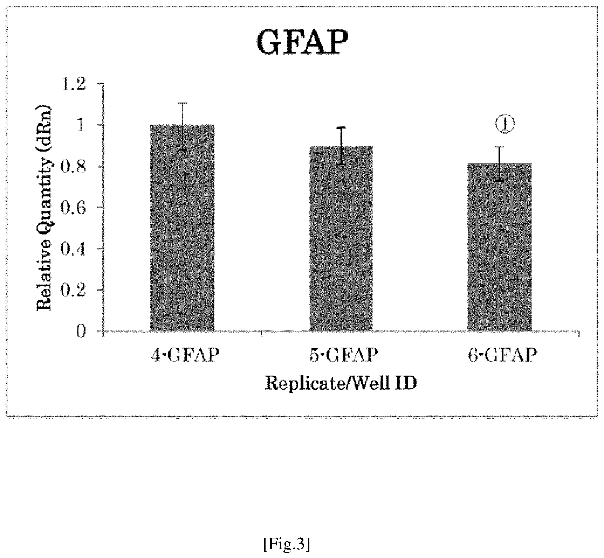

Results are shown in Fig.3.

Fig.3 is a graph as a drawing, which shows the expression of GFAP mRNA in Example 4. As shown in Fig.3, the expression of GFAP (GFAP mRNA/GAPDH mRNA) slightly increased from the level before the differentiation by changing a culture medium to the differentiation medium. This result indicates that the proliferation medium of the present invention maintains neural stem cells and the expression of GFAP therein.

### [Example 5]

### (Study of expression of CD antigen with flow cytometry)

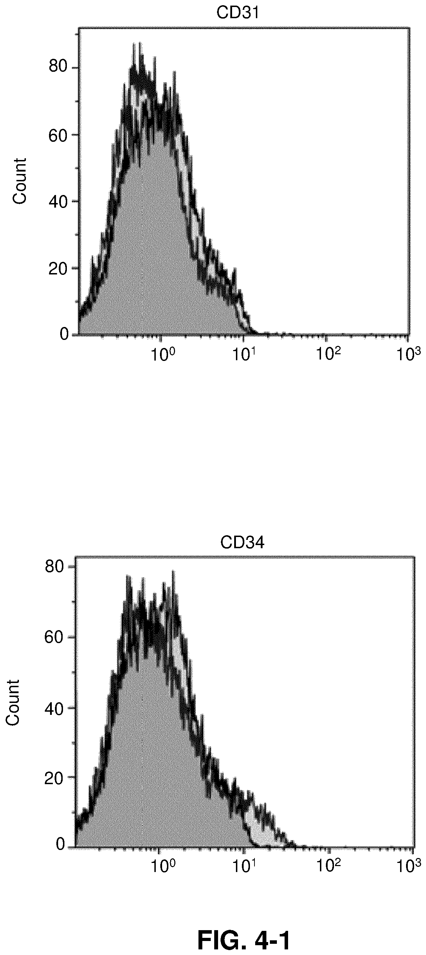

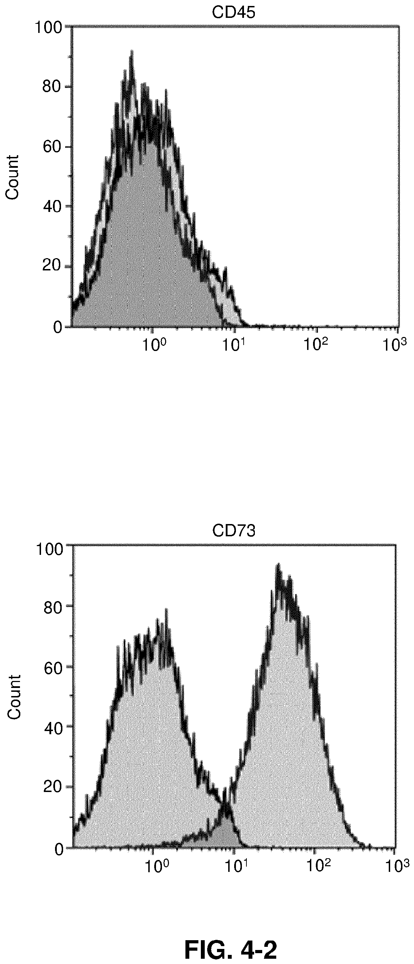

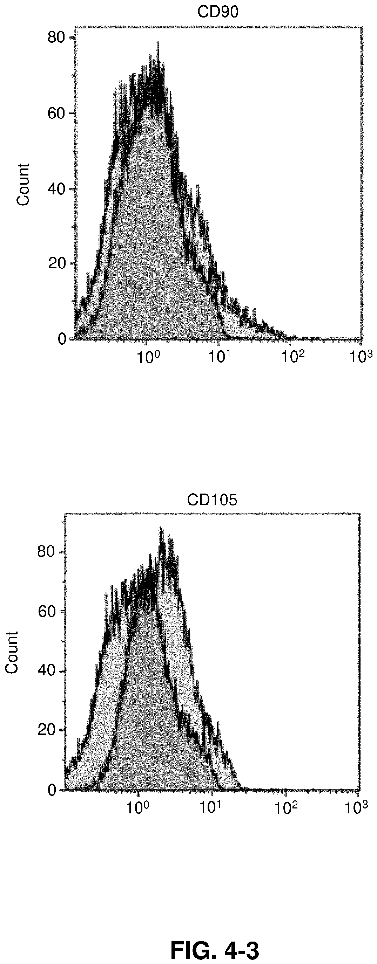

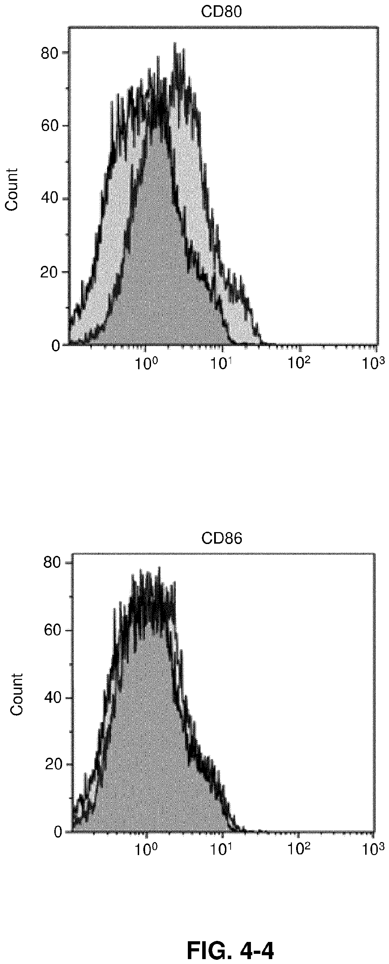

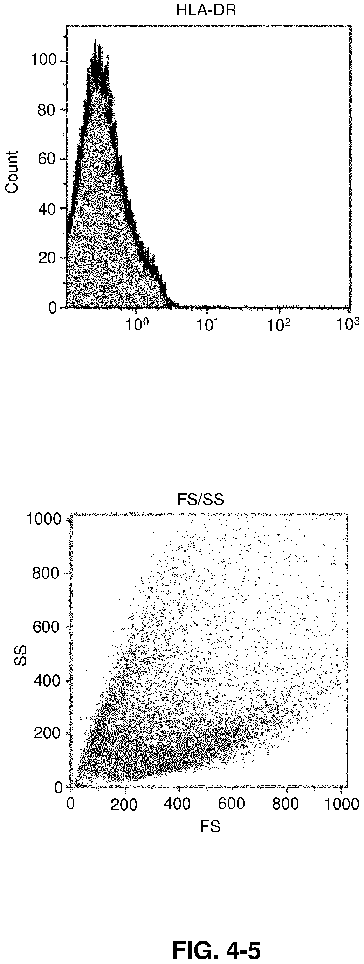

The cells cultured to be confluent were detached from the culture dish and isolated by using trypsin-EDTA solution. Then, the cells were blocked by being placed in a 3% BSA/PBS(-) solution for 30 minutes to be prevented from non-specific adsorption. After being fixed with 4% paraformaldehyde for 10 minutes, the cells were then incubated in 0.5% Triton X-100/PBS(-) at 4 °C for 5 minutes. The cells were then incubated at 4 °C overnight with a monoclonal antibody to CD73, CD73-PE (BD 561014). Subsequently, the cells were washed for 5 times to remove unbound antibodies and the expression of the antigen was analyzed with flow cytometry using EC800 cell analyzer from SONY Co. The results are shown in Fig.4.

Fig.4 is a graph as a drawing, which shows the expression of CD73 (CD73 expression profile) in Example 5. As shown in Fig.4, the cell was CD13-, CD34-, and CD45-negative, indicating that the cell was non-blood cell. The cell was CD90-negative, either, indicating that the cell was not human skin-derived mesenchymal stem cell. The strong expression of CD73 was the most prominent characteristic of CD expression profile of the cell.

### [Example 6]

### (Study of an effect on rat spinal cord injury model by BBB Score method)

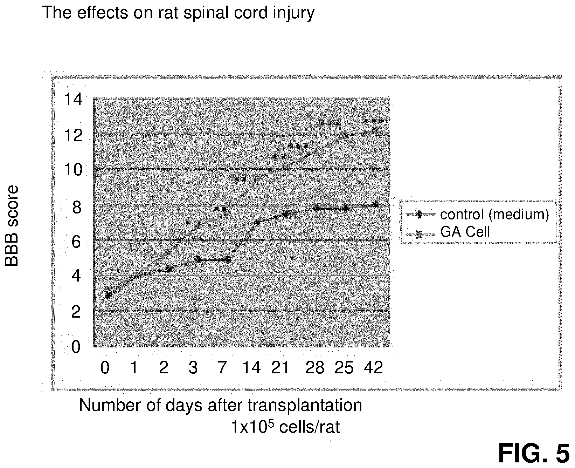

The cultured cells, which had not been treated to increase GDNF with SAG or the like, were detached from the culture dish using the enzyme, Accutase (GIBCO A11105). 1x10⁵ of the detached cells were suspended in 200 uL of a culture medium solution and transplanted by injecting into a spinal cord injured site of a rat of which spinal cord had been injured by weight-drop and an immunosuppressant had been administered to the injured site. A rat as a control was subjected to a sham operation of injecting the same amount of the culture medium only. In the experiment, the cell-transplanted group consisted of 5 rats and the control group consisted of 4 rats. An effectiveness of treating spinal injury was measured by BBB Score method where movements of an animal is observed with video equipment and rated. The results are shown in Fig.5. In the figure, the horizontal axis shows the number of days after the transplantation, and the longitudinal axis shows BBB scores. The full line shows the results of the transplanted group, the broken line shows the results of the control group, and the asterisks show results of a significant difference test by Student's T test with * indicating P<0.05, ** indicating p<0.01 and *** indicating p<0.001. From 3 days after the transplantation, the cell-transplanted group showed a statistically significant effect (p<0.05) compared with the control group by BBB Score difference of 2 points. From 35 days after the transplantation, the cell-transplanted group showed a statistically significant effect (p<0.001) compared with the control group by BBB Score difference of 4 points. In the report on a bone marrow stromal cell transplantation (Non-patent document 23), it took nearly 2 weeks to achieve a significant difference. In the iPS cell-derived neural stem cell transplantation (Non-patent document 1), it took 3 weeks to achieve a significant difference. Compared with these studies, the present result showed dramatically faster effects observed only after 3 days. Further, in the bone marrow stromal cell transplantation, only a significant difference with p<0.05 was observed even after 4 weeks, whereas, in the present invention, a superior significant difference with p<0.001 was observed after the same lapse of time. This result indicates that the cell obtained in the present invention is extremely effective for treating spinal cord injury.

### [Example 7]

### (Evaluation of tumorigenicity)

### Animal and Test Method

For the experiment, NOD/Shi-scid, IL-2Rγnull (trade mark) mice (6 weeks old female from Central Institute for Experimental Animals) were used. After 1-week of acclimatization, the mice were divided into 3 groups using a general-purpose grouping system (Visions Inc.) so that the groups have average body weight as equal as possible. In a right quadrant of the abdomen the mice of each group, cells cultured in the same manner as in Example 1 (hereinafter referred to as "cell A"), the culture medium as a negative control, or HeLa S3 (DS Pharma Biomedical Co.Ltd.) as a positive control was transplanted (each in an amount of 0.2 mL/individual) respectively by injection with a syringe (Myjector (trade mark), 27G). The groups are referred to as "cell A group (10 mice)", "culture medium group (10 mice)", and "HeLa S3 group (5 mice)", respectively. Three lines of the cell A were cultured in the same manner as in Example 1. Each line of cells were suspended in α-MEM medium at 1x10⁷ cells/mL and dispensed in 2-mL cryotubes in such a manner that no air is entrapped. About 3 hours after the dispensation, α-MEM medium was removed, and the cells were suspended again in a culture medium. The same number of 3-line cells were mixed and a suspension at a concentration of 5x10 ⁷ cells/mL was prepared, which was kept at 4 °C until the transplantation was performed within 2 hours after the preparation. A survival rate after the transplantation of the specimen, which was the mixture of the 3 lines of the cells, was 75 % of that measured just before the transplantation. The mice of each group were bred for 13 weeks and general conditions observations, body weight measurements, observation and size measurements of nodule were performed once a week according to "Standard operation manual for care and management of experimental animals" published from the Central Institute for Experimental Animals. In observing the nodule, a site where the specimen was transplanted was palpated with a finger. Presuming that formation of a nodule was detected in case a hardness to the touch was felt by finger, a major axis (L) and a minor axis (W) thereof were measured with vernier calipers. A volume of the nodule (V) was calculated by using a simple formula of tumor volume: V=LW²/2 (L and W in mm, V in mm³) according to "The Nude Mouse and Anticancer Drug Evaluation" (edited by T. Noguchi, Y.Sakurai. and M.Inaba, Kanishobo, June 1991, Kanishobo). The individual bodies of HeLa S3 group were euthanized at 5 to 8 weeks as humane endpoint of the observation because it was confirmed that a weight of nodule (calculated from a volume thereof assuming that a specific gravity being 1) exceeded 1/10 of the body weight during the observation period. The euthanization was performed by exsanguinating the mice under isoflurane anesthesia and the various organs in the chest cavity and the abdominal cavity were observed. Further, the skin at the transplanted site including the subcutaneous tissue was sampled and fixed with a 10% neutral buffered formalin solution. The formalin-fixed specimen was embedded in paraffin, which was sectioned and then stained with HE, and anti-HLA immunostaining followed by observation with an optical microscope.

### Result

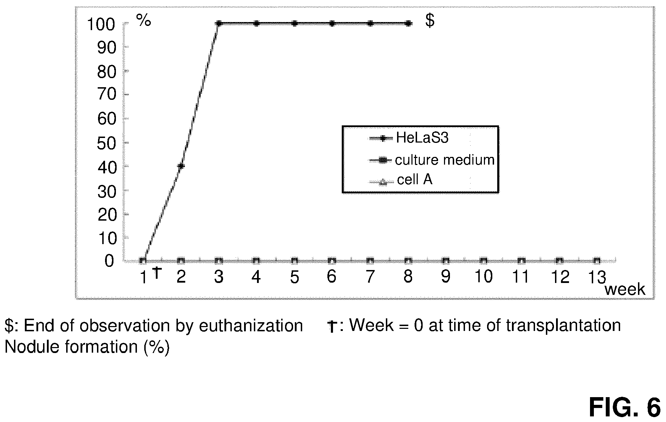

The results are shown in Fig.6. Fig.6 is a graph as a drawing, which shows an evaluation of carcinogenicity. In HeLa S3 group, a nodule was observed in 100 % of the individuals (5/5 cases) 3 weeks after the transplantation while no nodule was observed in the culture medium group and the cell A group until the end of the observation. In all the cases in HeLa S3 group, a nodule was observed. Because the nodule showed continuous increase in its volume and showed poorly differentiated adenocarcinoma histology as well as anti-HLA reaction, it was considered to be a tumor derived from HeLa S3 cell. In the cell A group, a histopathological examination revealed anti-HLA test positive fibroplasia in 6/10 cases, which was considered to be remaining transplanted cells. However, no nodule was observed in all cases over the whole test period, and no hyperplasia or neoplastic change was observed in HE stained histopathological images. These results indicate that the cell A does not show tumorigenicity under this test conditions.

### [Example 8]

### (Study of effects on human with spinal cord injury: Effects of GDNF)

Autologous cells from each patient's skin were cultured without treating SAG or the like and then detached from culture dishes with an enzyme, Accutase (GIBCO A11105). Prior to the transplantation, 1 mL of spinal fluid was taken by lumbar puncture. 1 x 10⁷ detached cells which were suspended in 1 mL of injectable physiological saline were then autologously transplanted at the punctured site. These 7 patients of cases 1-7 consisted of 6 males and 1 female with ages of from 32 to 51. The number of administrations were 2 to 3 with dose interval of from 1 to 3 months. The period from the injury to the administration ranged from 3 to 23 years. The patients were quadriplegic with C4-7 cervical cord injury. Side effects observed were headache and fever on the day of the administration. Among 7 patients, no therapeutic effects were observed in 3 patients. In the other 4 patients, therapeutic effects and subjective symptoms such as sweating in the arm, cutaneous sensation, improved urinary retention, increased grip strength, improved finger movements, or the like were observed. Only in the case 8, the cells with increased GDNF mRNA by SAG treatment were transplanted. the same number of cells as mentioned above were administered 2 times within 1 month. The patient of the case 8 was 47-year old male with Th3 thoracic cord injury with leg paralysis, and the period from the injury to the administration was 2 and a half years. A side effect of headache was observed on the day of the administration. The therapeutic effect became apparent one month after the administration, that is, he became capable of walking in an upright posture with an aid. Although the injury of the patient was milder as leg paralysis and the period to the administration was shorter from the injury with the case 8 compared with cases 1-7, the therapeutic effect was dramatic with a faster recovery.

### [Industrial Applicability]

The present invention can be utilized in the medical industry.

### [Sequence Listing Free Texts]

SEQ ID No.2: primer
SEQ ID No.3: primer
SEQ ID No.4: primer
SEQ ID No.5: primer
SEQ ID No.6: primer

## Claims

1. A method of producing a cultured cell comprising glial cell line-derived neurotrophic factor (GDNF) mRNA and CD73 protein, comprising the step of culturing a human skin-derived stem cell in a serum-free medium comprising SAG.

2. The method according to claim 1, wherein the serum-free medium further comprises one or both of purmorphamine and sonic hedgehog (SHH) protein.

3. The method according to claim 1, wherein the serum-free medium further comprises B-27 supplement.

4. The method according to claim 1, wherein the serum-free medium further comprises a ROCK inhibitor.

5. The method according to claim 1, wherein the serum-free medium further comprises EGF or FGF2.

6. The method according to claim 1, wherein the serum-free medium comprises calcium ion at a concentration of from 0.03 mM to 0.12 mM.

7. The method according to claim 1, wherein the human skin-derived stem cell is obtained by treating a collected human skin with dispase, trypsin, and collagenase in this order.

8. A method of producing a therapeutic agent for spinal cord injury comprising the steps of producing a cultured cell by the method according to claim 1, and
wherein the therapeutic agent comprises at least one of the cultured cell, a medium obtained in the previous step, and a secretion obtained in the previous step.
